# FASCICULE DE BREVET EUROPEEN

(11) **EP 2 203 455 B1**
(45) Date de publication et mention de la délivrance du brevet: **30.08.2017**
(21) Numéro de dépôt: 08838681.8
(22) Date de dépôt: 23.09.2008
(51) Int. Cl.: C07D 493/04

(54) **PROCEDE DE SYNTHESE DE DERIVES ANTICANCEREUX DE (POLY)AMINOALKYLAMINOACETAMIDE D'EPIPODOPHYLLOTOXINE**
VERFAHREN ZUR SYNTHESE VON ANTIKREBS- (POLY)AMINOALKYLAMINOACETAMID-DERIVATEN AUS EPIPODOPHYLLOTOXIN
METHOD FOR THE SYNTHESIS OF ANTICANCER (POLY)AMINOALKYLAMINOACETAMIDE DERIVATIVES OF EPIPODOPHYLLOTOXIN

(30) Priorité: 25.09.2007 FR 0706692
(43) Date de publication de la demande: 07.07.2010
(73) Titulaire: Pierre Fabre Médicament, 92100 Boulogne (FR)
(72) Inventeur: GUMINSKI, Yves, F-81090 Lagarrigue (FR); GROUSSEAUD, Martial, F-81100 Castres (FR); IMBERT, Thierry, F-81290 Viviers Les Montagnes (FR)
(74) Mandataire: Regimbeau
(86) Numéro de dépôt international: PCT/FR2008/051697
(87) Numéro de publication internationale: WO 2009/050363

(56) Documents cités:
- WO-A-97/21713
- WO-A-2004/073375
- WO-A-2005/100363
- WO-A-2007/010007

## Description

La présente invention a pour objet un nouveau procédé de préparation des dérivés (poly)aminoalkylaminoacétamide d'épipodophyllotoxine de formule 1, et de leurs sels pharmaceutiquement acceptables. 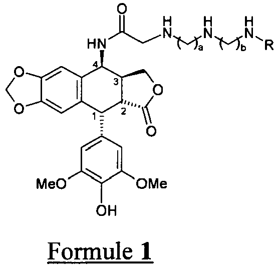 dans laquelle R représente un atome d'hydrogène ou un groupe -(CH₂)_{c}-NH₂ avec 2 ≤ a,b,c ≤ 5.
Ces composés sont constitués par une partie lignane de type épipodophyllotoxine et d'une partie polyamine greffée sur la position 4 de l'épipodophyllotoxine avec un motif acétamide.
La présence de la chaîne polyamine confère à la molécule ses propriétés d'hydrosolubilité particulièrement pour ses chlorhydrates, ainsi que des propriétés pharmacologiques particulièrement intéressantes dans le traitement des cancers.
Ces composés, décrits dans la demande WO 2005/100363, sont des composés anticancéreux, particulièrement utiles dans le traitement des tumeurs solides ou non solides tels les mélanomes, les cancers colorectaux, les cancers du poumon, prostate, vessie, sein, utérus, estomac, pancréas, foie, ovaires ainsi que dans le traitement des leucémies, lymphomes et myélomes, les cancers de la sphère ORL et les tumeurs du cerveau.
Le procédé de synthèse décrit dans WO 2005/100363, pour préparer les composés de formule **1** utilise comme produit de départ la podophyllotoxine de formule **2** : 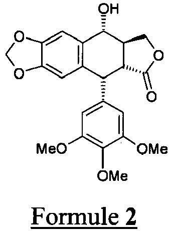 puis la 4'- déméthylépipodophyllotoxine de formule 3 : 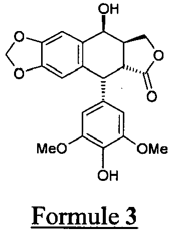 sur laquelle on fait réagir le chloroacétonitrile en milieu acide pour obtenir l'intermédiaire de synthèse 4-chloroacétamido-4'-déméthylépipodophyllotoxine de formule 4 : 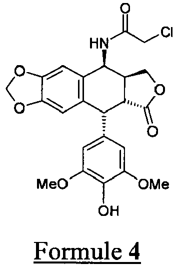 Ce composé est ensuite condensé avec un réactif aminé primaire de formule 5 : 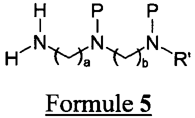 dans laquelle R' représente un hydrogène ou une chaîne -(CH₂)_{c}-NHP et où P représente un groupement protégeant les fonctions amine.

Les groupements protecteurs appropriés peuvent être un radical : benzyl, benzyloxycarbonyl ou tert-butyloxycarbonyl. Cette condensation se fait dans un mélange de solvants comprenant un solvant polaire aprotique (acétonitrile, DMF) en présence d'une base de Lewis (triéthylamine).

Cependant ce procédé, outre le fait qu'il comporte un nombre d'étapes élevé et donc un rendement global assez faible, présente deux inconvénients :

D'une part, les conditions utilisées dans le brevet WO 2005/100363 sont propices à l'épimérisation du carbone en position 2 du dérivé de l'épipodophyllotoxine de formule 1, conduisant à une forme cis-lactone, nommée « picro » de formule 7 :

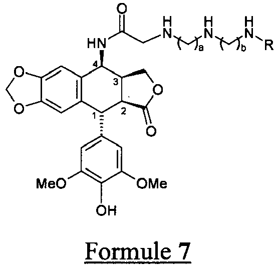

La purification du produit trans lactone recherché est donc difficile et nécessite des opérations de chromatographies laborieuses et coûteuses.
D'autre part, la méthode décrite ci-dessus fournit également des sous-produits de type bis alkylation, par réaction d'une autre molécule de 4β-chloroacétamido-4'-déméthylépipodophyllotoxine sur le produit de formule **1** déjà formé. L'utilisation d'un excès de réactif aminé primaire de formule **5** est alors nécessaire pour une transformation la plus complète des matières premières tout en minimisant l'obtention de produits secondaires ce qui nécessite une étape difficile de récupération de l'amine en excès, rendant ce procédé peu économique.

De manière inattendue, la demanderesse a constaté qu'en utilisant un réactif aminé primaire, répondant à la formule **6** dans laquelle a, b et R ont la même signification que précédemment, mais dans lequel les fonctions amines ne sont pas protégées, les produits secondaires potentiels sont minimisés et leur présence ne présente pas d'handicap majeur à l'obtention d'un composé pur final.

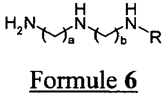

La condensation directe de la 4-chloroacétamido-4'-déméthylépipodophyllotoxine de formule **4** avec le réactif aminé primaire, sans étape supplémentaire de protection des fonctions amine de ce dernier, se fait alors dans des conditions satisfaisantes en terme de rendement et de pureté du produit obtenu. Ainsi, dans le cas du composé pour lequel a = 3, b = 4, c = 3, on passe d'une synthèse comprenant 11 étapes, lorsque l'on met en oeuvre le procédé tel que décrit dans WO 2005/100363, avec un rendement global d'environ 15% ; à une synthèse, objet de la présente invention, qui permet en 3 étapes seulement d'obtenir un rendement global de 30%.
Dans le cadre de la synthèse objet de la présente invention, il est mis en oeuvre une quantité stoechiométrique des réactifs, ce qui en minimise le coût.
Le produit prépondérant de la réaction est alors le produit d'alkylation formant le composé à chaîne linéaire par substitution de l'amine primaire de façon très majoritaire. Une telle sélectivité de réactivité est surprenante compte-tenu des produits secondaires obtenus en utilisant le voie de synthèse décrite dans WO 2005/100363. L'utilisation de l'amine non protégée conduit majoritairement au produit recherché.
Cette méthode présente par ailleurs l'avantage de limiter le nombre d'étapes du fait que les étapes de protection du réactif aminé primaire ne sont plus nécessaires.

La présente invention concerne donc un procédé de synthèse des composés de formule **1** comprenant une étape de condensation de l'intermédiaire 4β-chloroacétamido-4'-déméthylépipodophyllotoxine de formule **4** avec un réactif aminé primaire de formule **6** ne comportant pas de protection préalable des fonctions amines en utilisant une quantité stoechiométrique desdits réactifs 4 et 6.
Les composés de formule **1** sont obtenus de manière préférentielle sous forme de chlorhydrate.
Cette réaction de condensation se fait de manière directe sans qu'aucune fonction amine du réactif aminé primaire de formule **6** utilisé ne nécessite une protection par un groupement protecteur approprié quel qu'il soit.
De façon préférentielle l'étape de condensation de la 4β-chloroacétamido-4'-déméthylépipodophyllotoxine de formule **4** avec le réactif aminé primaire non protégé de formule **6** se fait dans un solvant polaire aprotique.

De manière préférentielle le solvant polaire aprotique utilisé lors de l'étape de condensation est choisi parmi: la diméthylformamide, la diméthylacétamide, la N-méthyl pyrrolidone ou encore le diméthylsulfoxide.

De manière préférentielle également, la réaction de condensation est réalisée dans une gamme de température comprise entre - 20° C et 30°C. Un échauffement est observé quand l'opération est menée sur une quantité de plusieurs dizaines de grammes et donc une maîtrise de la température de la réaction est préférable. De manière encore plus préférentielle, on maintiendra donc la température à 0°C.

L' invention concerne également un procédé de synthèse des composés de formule **1** dans lequel l'étape de condensation de l'intermédiaire 4β-chloroacétamido-4'-déméthylépipodophyllotoxine de formule **4** avec le réactif aminé primaire de formule **6** est suivie d'une étape de récupération du composé **1.**

L'étape de récupération du produit de formule **1** se fait, de manière préférentielle par précipitation dans un solvant alcoolique tel le méthanol ou l'éthanol, suivie d'une étape de chromatographie en phase inverse en milieu acide. Le composé est purifié dans une solution acide, comme l'acide chlorhydrique. Il n'est pas soumis au risque d'épimérisation au niveau de la lactone conduisant au dérivé « picro ». Une lyophilisation finale permet d'isoler le sel du composé recherché.

De manière préférentielle également, on utilise comme réactif aminé primaire non protégé de formule **6,** la spermine ou la spermidine de formules **8** et **9** suivantes.

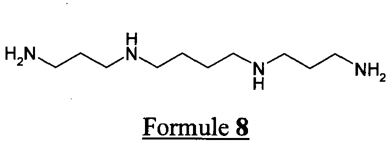

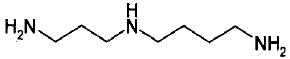

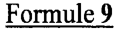

Dans le cas de la condensation avec la spermidine, polyamine dissymétrique, 2 composés isomères de formule **1** sont obtenus à parts égales (composés pour lesquels a = 3, b = 4, R = H et a = 4, b = 3, R = H).
Dans le cas de la condensation avec la spermine, c'est le composé pour lequel a = 3,
b = 4 et R = (CH₂)₃-NH₂ qui est obtenu.

La présente invention concerne donc également un procédé de synthèse du composé pour lequel a = 3, b = 4, R = H soit le 2-[3-(4-aminobutylamino)-propylamino]-N-[9-(4-hydroxy-3,5-diméthoxyphenyl)-8-oxo-5,5a,6,8,8a,9-hexahydrofuro[3',4':6,7]naphto[2,3-d][1,3]dioxol-5-yl]-acétamide comprenant une étape de condensation de la 4β-chloroacétamido-4'-déméthylépipodophyllotoxine de formule **4** avec la spermidine suivie d'une étape de récupération de ce composé.

La présente invention concerne également un procédé de synthèse du composé pour lequel a = 4, b = 3, R = H soit le 2-[4-(3-aminopropylamino)-butylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide comprenant une étape de condensation de la 4β-chloroacétamido-4'-déméthylépipodophyllotoxine de formule **4** avec la spermidine suivie d'une étape de récupération de ce composé.

La présente invention concerne également un procédé de synthèse du composé pour lequel a = 3, b = 4 et R = (CH₂)₃-NH₂ soit le 2-{3-[4-(3-aminopropylamino)-butylamino]-propylamino}-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide comprenant une étape de condensation de la 4β-chloroacétamido-4'-déméthylépipodophyllotoxine de formule **4** avec la spermine suivie d'une étape de récupération de ce composé.

L'invention porte également sur l'utilisation du réactif aminé primaire de formule **6** à la préparation des composés de formule générale **1** selon un procédé mettant en jeu une étape de condensation entre ce réactif aminé primaire et la 4β-chloroacétamido-4'-déméthylépipodophyllotoxine de formule **4.**

La présente invention concerne également un procédé de préparation des composés de formule **1** à partir de podophyllotoxine de formule **2,** comprenant les étapes suivantes et caractérisé par le fait que le réactif primaire aminé utilisé dans l'étape c) est le réactif de formule **6.** La condensation de l'étape c) se fait de manière directe sans étape de protection, par un quelconque groupement protecteur, des fonctions amine du réactif aminé primaire utilisé.
a) Préparation de la 4'-déméthylépipodophyllotoxine de formule **3** à partir de la podophyllotoxine de formule **2**
b) Transformation de la 4'-déméthylépipodophyllotoxine en 4β-chloroacétamido-4'-déméthylépipodophyllotoxine de formule **4**
c) Condensation de la 4β-chloroacétamido-4'-déméthylépipodophyllotoxine avec un réactif aminé primaire de formule **6**

L'étape a) se fera de manière préférentielle par le procédé décrit dans la demande de brevet WO 97/21713, c'est à dire par traitement de la podophyllotoxine par un couple acide fort-sulfure aliphatique, aromatique ou fonctionnalisé en présence d'un acide organique ou minéral ou bien en présence d'eau avec ou sans solvant organique miscible à l'eau.

L'étape b) se fera de manière préférentielle selon la méthode décrite dans la demande WO 2005/100363, c'est à dire par réaction de la 4'-déméthylépipodophyllotoxine obtenue à l'étape précédente avec le chloroacétonitrile en milieu acide.

L'étape c) ainsi que l'étape ultérieure de récupération sont réalisées comme décrit précédemment, le réactif aminé primaire utilisé ne comportant aucune protection sur ses fonctions amine.

A l'issu de l'étape de récupération, le composé de formule générale **1** peut éventuellement être salifié à l'aide d'un acide minéral ou organique.

De manière préférentielle l'étape de condensation c) est réalisée dans un solvant polaire aprotique choisi parmi: la diméthylformamide, la diméthylacétamide, la N-méthyl pyrrolidone ou encore le diméthylsulfoxide.

De manière préférentielle également, la réaction de condensation est réalisée dans une gamme de température comprise entre - 20° C et 30°C, plus particulièrement à 0°C.
L'étape de récupération du composé de formule **1** se fait, de manière préférentielle par précipitation dans un solvant alcoolique tel le méthanol ou l'éthanol, suivie d'une étape de chromatographie en phase inverse en milieu acide. Le composé est purifié dans une solution acide , comme l'acide chlorhydrique.

De manière préférentielle également le réactif aminé primaire non protégé de formule **6** utilisé à l'étape de condensation c) est la spermine ou la spermidine.
Lorsque c'est la spermidine qui est utilisée à l'étape c) on obtiendra les 2 composés isomères de formule **1** pour lesquels a = 3, b = 4, R = H et a = 4, b = 3, R = H en parts égales.
Dans le cas de la condensation avec la spermine, c'est le composé pour lequel a = 3, b = 4 et R = (CH₂)₃-NH₂ qui est obtenu.
L'invention porte également sur l'utilisation du réactif aminé primaire de formule 6 dans la préparation des composés de formule générale 1 à partir de la podophyllotoxine selon les étapes a) puis b) puis c) décrites précédemment.

La présente invention concerne également un procédé de synthèse du composé pour lequel a = 3, b = 4, R = H ou du composé pour lequel a = 4, b = 3 et R = H soit le 2-[3-(4-aminobutylamino)-propylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide ou le 2-[4-(3-aminopropylamino)-butylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide comprenant les étapes suivantes :
a) Préparation de la 4'-déméthylépipodophyllotoxine de formule **3** à partir de la podophyllotoxine de formule **2**
b) Transformation de la 4'-déméthylépipodophyllotoxine en 4β-chloroacétamido-4'-déméthylépipodophyllotoxine de formule **4**
c) Condensation de la 4β-chloroacétamido-4'-déméthylépipodophyllotoxine de formule **4** avec la spermidine.

Ces 3 étapes sont suivies d'une étape de récupération du 2-[3-(4-aminobutylamino)-propylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide ou du 2-[4-(3-aminopropylamino)-butylamino]-N-[9-(4-hydroxy-3,5-diméthoxypényl)-8-oxo-5,5a,6,8,8a,9-hexahydrofuro[3',4':6,7] naphto[2,3-d][1,3]dioxol-5-yl]-acétamide, ces produits étant éventuellement salifiés à l'aide d'un acide minéral ou organique.

La présente invention concerne également un procédé de synthèse du composé pour lequel a = 3, b = 4 et R = (CH₂)₃-NH₂ soit le 2-{3-[4-(3-aminopropylamino)-butylamino]-propylamino}-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide comprenant les étapes suivantes :
a) Préparation de la 4'-déméthylépipodophyllotoxine de formule **3** à partir de la podophyllotoxine de formule **2**
b) Transformation de la 4'-déméthylépipodophyllotoxine en 4β-chloroacétamido-4'-déméthylépipodophyllotoxine de formule **4**
c) Condensation de la 4β-chloroacétamido-4'-déméthylépipodophyllotoxine de formule **4** avec la spermine.
suivies d'une étape de récupération du 2-{3-[4-(3-aminopropylamino)-butylamino]-propylamino}-N-[9-(4-hydroxy-3,5-diméthoxyphényl)-8-oxo-5,5a,6,8,8a,9-hexahydrofuro[3',4':6,7] naphto[2,3-d][1,3]dioxol-5-yl]-acétamide et éventuellement d'une salification.

Les exemples qui suivent illustrent l'invention sans en limiter la portée.

### Exemple 1 :

### Synthèse du composé de formule 1 avec a = 3, b =4, c = 3 soit le 2-{3-[4-(3-aminopropylamino)-butylamino]-propylamino}-N-[9-(4-hydroxy-3,5-diméthoxyphenyl)-8-oxo-5,5a,6,8,8a,9-hexahydrofuro[3',4':6,7]naphto[2,3-d][1,3]dioxol-5-yl]-acétamide, sous forme de chlorhydrate à partir de la 4β-chloroacétamido-4'-déméthylépipodophyllotoxine et de spermine aux fonctions amine non protégées

Le schéma de la synthèse est le suivant :

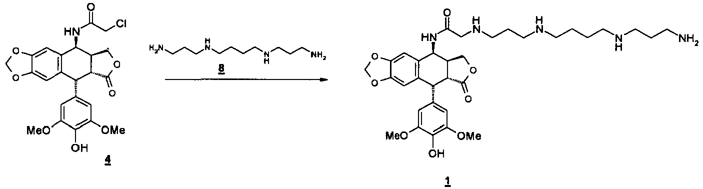

A 1 g (2.1 mmol) de 4β-chloroacétamido-4'-déméthylépipodophyllotoxine, en solution dans 5 mL de DMF , est additionné 0.43 g (2.1 mmol) de spermine de formule 8 dans 5 mL DMF. L'agitation est maintenue 5h. On ajoute alors 20 mL d'EtOH , puis une solution d'isopropanol/HCl est alors additionnée, jusqu'à pH légèrement acide. Le chlorhydrate précipite. Les cristaux sont filtrés et séchés pour obtenir 1.9 g de composé **1** brut. On réalise une purification par HPLC préparative (Lichrospher 100 RP 18, élution HCl : c = 5mM). Les fractions sont lyophilisées puis reprises par de l'éther éthylique et le chlorhydrate du composé **1** est obtenu sous forme amorphe, avec une pureté de 97% rendement 40%.
F°C : 267°C. ¹H NMR: (DMSO) δ 9.07(d, 1H, J = 8.32 Hz, NHCO), 8.27(s, 1H, ArOH), 6.80(s, 1H, H₅), 6,55(s, 1H, H₈), 6,23(s, 2H, H_{2'}, H_{6'}), 6.01 (d, 2H, J = 12 Hz, OCH₂O), 5,23(dd, 1H, J = 5.3 and 8.1 Hz, H₄), 4.52(d, 1H, J = 5,2 Hz, H₁), 4,28(t, 1H, J = 8 Hz, H₁₁ₐ), 3,94(dd, 1H, J = 8.8 and 10.4 Hz, H_{11b}), 3.8(m, 2H, CH₂CO), 3,63(s, 6H, 2xOCH₃), 3.22(dd, 1 H, j = 5 and 14.4 Hz, H₂), 3.06(m, 3H, H₃ and CH₂NH), 2.99(m, 4H, CH₂NH), 2.89(m, 6H, CH₂NH), 2.08(t, J = 7.6 Hz, 2H, sat. CH₂), 1.99(q, 2H, J = 7.2 Hz, sat. CH₂), 1.73(m, 4H, sat. CH₂). MS-ESI (m/z) 642.2 (MH+). Anal: C₃₃H₄₇N₅O₈, 4HCl, calc. C% 50.32, H% 6.53, N% 8.89. trouvé C% 50.264, H% 6.57, N% 8.66.

### Exemple 2 :

### Procédé de préparation du composé de formule 1 avec a= 3 , b= 4 , R= H, soit le 2-[3-(4-aminobutylamino)-propylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide, chlorhydrate et le composé de formule 1 et a= 4 , b= 3 , R= H soit le 2-[4-(3-aminopropylamino)-butylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide, sous forme de chlorhydrate à partir de 4β-chloroacétamido-4'-déméthylépipodophyllotoxine et de spermidine aux fonctions amine non protégées

Le schéma de cette synthèse est le suivant :

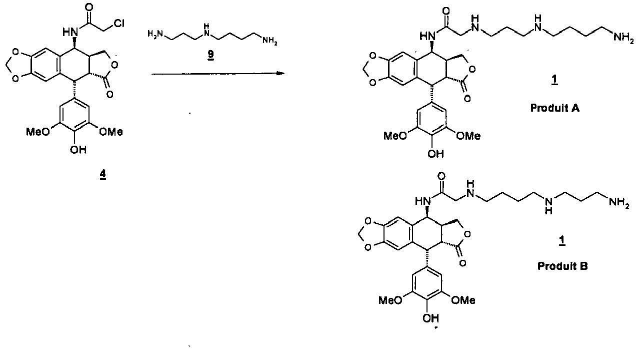

Dans les mêmes conditions que pour le dérivé de l'exemple 1, mais en remplaçant la spermine par la spermidine de formule **9,** on obtient les composés de formule **1** (produit A : a = 3, b = 4, R = H ; et produit B : a = 4, b = 3, R = H). De façon identique à l'exemple 1, on isole ces 2 produits en proportions équivalentes, avec un rendement global de 40%.
Ces produits sont en tous points identiques aux composés obtenus respectivement dans les exemples 31 et 32 de la demande de brevet WO 2005/100363.

Ces exemples 1 et 2 sont transposables à la synthèse de tous les composés de la formule **1** en utilisant au lieu de la spermine ou de la spermidine , le réactif aminé primaire non protégé de formule **6** correspondant.

### Exemple 3 :

### Synthèse du composé de formule 1 avec a = 3, b = 4, c = 3 soit le 2-{3-[4-(3-aminopropylamino)-butylamino]-propylamino}-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide, sous forme de chlorhydrate à partir de la podophyllotoxine en 3 étapes

### 1^{er} stade

Le schéma de synthèse est le suivant :

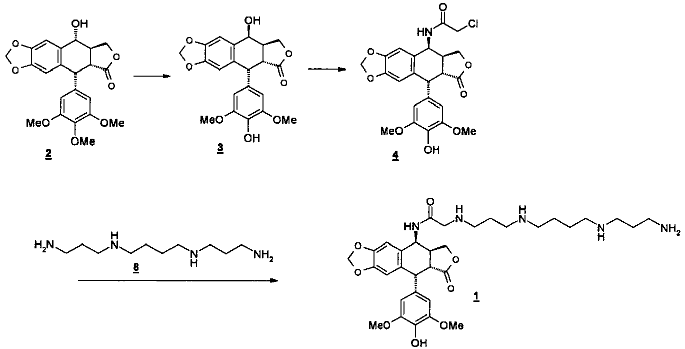

10 g (24 nmol) de podophyllotoxine sont dissous dans 60 ml d'acide trifluoroacétique. On ajoute successivement 5,4 ml (72 mmol) d'acide méthane sulfonique. L'agitation est maintenue pendant 9h, 5,4 ml (72 mmol) de diméthylsulfure sont à nouveau ajoutés et l'agitation est maintenue pendant 9h. Le milieu est jeté sur de la glace (600ml) et extrait par de l'acétate d'éthyle (3 x 300ml). Les phases organiques sont lavées à l'eau puis avec une solution de NaHCO₃ jusqu'à neutralité. Après séchage sur sulfate de sodium, filtration et évaporation, on obtient 6,3 g de 4'- déméthylépipodophyllotoxine , engagés directement au stade suivant.

### 2^{ème} stade

On additionne 30 g de 4'-déméthylépipodophyllotoxine à 47,4 ml de chloroacétonitrile, puis sous agitation, on ajoute 3 gouttes d'acide sulfurique concentré. L'agitation est maintenue 3h à température ambiante. On ajoute alors 300 ml d'isopropanol sous agitation. Le précipité obtenu est filtré et lavé avec 200 ml d'isopropanol. On rince le précipité avec de l'eau jusqu'à pH neutre, puis à l'éther éthylique. On obtient après séchage sous vide 34,2 g (rendement 96%) d'un solide blanc (PF = 240° C) correspondant à la 4β-chloroacétamido-4'-déméthylépipodophyllotoxine.

### 3^{ème} stade

A partir de la 4β-chloroacétamido-4'-déméthylépipodophyllotoxine obtenue à l'étape précédente, la synthèse est poursuivie selon la méthode décrite à l'exemple 1 pour obtenir le produit de formule **1** (a = 3, b = 4, c = 3) .

Cet exemple est transposable à tous les composés de formule **1** en utilisant les réactifs aminés primaires non protégés de formule **6** correspondants.

## Revendications

1. Procédé de préparation des dérivés (poly)aminoalkylaminoacétamide d'épipodophyllotoxine de formule **1** et de leurs sels pharmaceutiquement acceptables, dans laquelle R représente un atome d'hydrogène ou un groupe -(CH₂)_{c}-NH₂ avec 2 ≤ a,b,c ≤ 5. 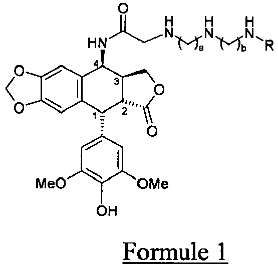 **caractérisé en ce qu'**il comprend une étape de condensation de la 4β-chloroacétamido-4'-déméthylépipodophyllotoxine de formule **4** 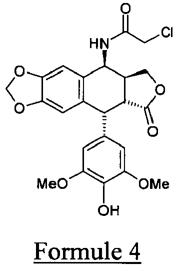 avec un réactif aminé primaire de formule **6:** a, b, c et R ayant les mêmes significations que précédemment et ne comportant pas de protection préalable des fonctions amines 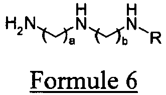 **Caractérisé en ce que** ladite étape de condensation se fait dans un solvant aprotique polaire et dans une gamme de température comprise entre -20°C et +30°C, et que lesdits réactifs de formule 4 et 6 sont en quantité stoechiométrique.

2. Procédé de préparation des dérivés (poly)aminoalkylaminoacétamide d'épipodophyllotoxine de formule **1** selon la revendication 1 **caractérisé en ce qu'**ils sont obtenus sous forme de chlorhydrate.

3. Procédé selon l'une quelconque des revendications 1 à 2 **caractérisé en ce que** le solvant polaire aprotique est choisi parmi le diméthylformamide, le diméthylacétamide, la N-méthylpyrrolidone ou le diméthylsulfoxide.

4. Procédé selon l'une quelconque des revendications 1 à 3 **caractérisé en ce que** la réaction de condensation entre la 4β-chloroacétamido-4'-déméthylépipodophyllotoxine et le réactif aminé primaire de formule **6** se fait à la température de 0°C.

5. Procédé selon l'une quelconque des revendications 1 à 4 **caractérisé en ce que** la réaction de condensation entre la 4β-chloroacétamido-4'-déméthylépipodophyllotoxine et le réactif aminé primaire de formule **6** est suivie d'une étape de récupération du produit de formule générale **1.**

6. Procédé selon l'une quelconque des revendications 1 à 5 **caractérisé en ce que** la récupération du composé de formule 1 se fait par précipitation dans un solvant alcoolique, suivi d'une chromatographie en phase inverse en milieu acide.

7. Procédé selon l'une quelconque des revendications 1 à 6 **caractérisé en ce que** le réactif aminé primaire de formule générale **6** utilisé lors de l'étape de condensation avec la 4β-chloroacétamido-4'-déméthylépipodophyllotoxine est la spermine ou la spermidine.

8. Procédé de préparation du composé de formule **1** dans lequel a = 3, b = 4 et c = 3 soit le 2-{3-[4-(3-aminopropylamino)-butylamino]-propylamino}-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide selon l'une quelconque des revendications 1 à 7 **caractérisé en ce qu'**il comprend une étape de condensation de la 4β-chloroacétamido-4'-déméthylépipodophyllotoxine avec la spermine.

9. Procédé de préparation du composé de formule **1** pour lequel a = 3, b = 4, R = H soit le 2-[3-(4-aminobutylamino)-propylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide selon l'une quelconque des revendications 1 à 7 **caractérisé en ce qu'**il comprend une étape de condensation de la 4β-chloroacétamido-4'-déméthylépipodophyllotoxine de formule **4** avec la spermidine.

10. Procédé de préparation du composé de formule **1** pour lequel a = 4, b = 3, R = H soit le 2-[4-(3-aminopropylamino)-butylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide selon l'une quelconque des revendications 1 à 7 **caractérisé en ce qu'**il comprend une étape de condensation de la 4β-chloroacétamido-4'-déméthylépipodophyllotoxine de formule **4** avec la spermidine.

11. Procédé de préparation des composés de formule **1** selon les revendications 1 à 7 **caractérisé en ce qu'**il comprend les étapes suivantes :
**a)** Préparation de la 4'-déméthylépipodophyllotoxine de formule **3** à partir de la podophyllotoxine de formule **2** 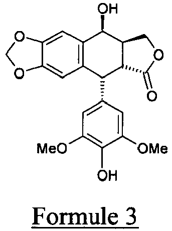 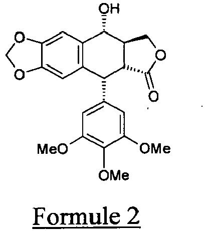
**b)** Transformation de la 4'-déméthylépipodophyllotoxine de formule **3** obtenue à l'étape a) en 4β-chloroacétamido-4'-déméthylépipodophyllotoxine de formule **4** 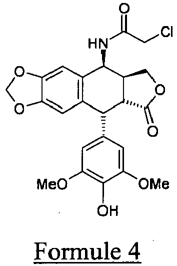
c) Condensation de la 4β-chloroacétamido-4'-déméthylépipodophyllotoxine obtenue à l'étape b) avec un réactif aminé primaire de formule **6.**

12. Procédé de préparation du composé de formule **1** dans lequel a = 3, b = 4 et c = 3 soit le 2-{3-[4-(3-aminopropylamino)-butylamino]-propylamino}-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide selon la revendication 11 **caractérisé en ce qu'**il comprend les étapes suivantes :
a) Préparation de la 4'-déméthylépipodophyllotoxine à partir de la podophyllotoxine
b) Transformation de la 4'-déméthylépipodophyllotoxine obtenue à l'étape a) en 4β-chloroacétamido-4'-déméthylépipodophyllotoxine
c) Condensation de la 4β-chloroacétamido-4'-déméthylépipodophyllotoxine obtenue à l'étape b) avec la spermine.

13. Procédé de préparation du composé de formule 1 dans lequel a = 3, b = 4 et R = H soit le 2-[3-(4-aminobutylamino)-propylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide selon la revendication 11 **caractérisé en ce qu'**il comprend les étapes suivantes :
a) Préparation de la 4'-déméthylépipodophyllotoxine à partir de la podophyllotoxine
b) Transformation de la 4'-déméthylépipodophyllotoxine obtenue à l'étape a) en 4β-chloroacétamido-4'-déméthylépipodophyllotoxine
c) Condensation de la 4β-chloroacétamido-4'-déméthylépipodophyllotoxine obtenue à l'étape b) avec la spermidine.

14. Procédé de préparation du composé de formule **1** dans lequel a = 4, b = 3 et R = H soit le 2-[4-(3-aminopropylamino)-butylamino]-N-[9-(4-hydroxy-3,5-diméthoxyphényl)-8-oxo-5,5a,6,8,8a,9-hexahydrofuro[3',4':6,7]naphto[2,3-d][1,3]dioxol-5-yl]-acétamide selon la revendication 11**caractérisé en ce qu'**il comprend les étapes suivantes :
a) Préparation de la 4'-déméthylépipodophyllotoxine à partir de la podophyllotoxine
b) Transformation de la 4'-déméthylépipodophyllotoxine obtenue à l'étape a) en 4β-chloroacétamido-4'-déméthylépipodophyllotoxine
c) Condensation de la 4β-chloroacétamido-4'-déméthylépipodophyllotoxine obtenue à l'étape b) avec la spermidine .

15. Utilisation des composés de formule générale **6** pour la préparation des composés de formule générale **1** selon les revendications 1 à 7

16. Utilisation des composés de formule générale **6** pour la préparation des composés de formule générale **1** selon les revendications 11 à 14.

## Patentansprüche

1. Verfahren zur Herstellung von (Poly)aminoalkylaminoacetamid-Derivaten von Epidophyllotoxin der Formel **1** und deren pharmazeutisch akzeptablen Salzen, in der R für ein Wasserstoffatom oder eine Gruppe -(CH₂)_{c}-NH₂ steht, wobei 2 ≤ a, b, c ≤ 5 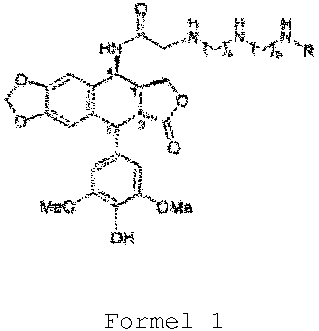 **dadurch gekennzeichnet, dass** das Verfahren einen Schritt der Kondensation des 4β-Chloracetamido-4'-demethylepipodophyllotoxins der Formel **4** 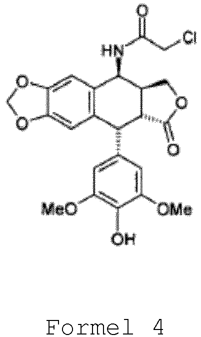 mit einem primären Aminreaktanten der Formel **6** umfasst; wobei a, b, c und R dieselben Bedeutungen wie zuvor aufweisen und kein vorheriges Schützen der Aminfunktionen umfassen 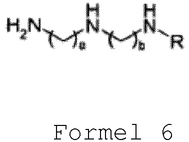 **dadurch gekennzeichnet, dass** der besagte Kondensationsschritt in einem aprotischen polaren Lösungsmittel und in einem Temperaturbereich, der zwischen -20°C und +30°C liegt, durchgeführt wird und die besagten Reaktanten der Formeln 4 und 6 in einer stöchiometrischen Menge sind.

2. Verfahren zur Herstellung von (Poly)aminoalkylaminoacetamid-Derivaten von Epidophyllotoxin der Formel **1** nach Anspruch 1, **dadurch gekennzeichnet, dass** sie in der Form eines Hydrochlorids erhalten werden.

3. Verfahren nach einem der Ansprüche 1 bis 2, **dadurch gekennzeichnet, dass** das polare aprotische Lösungsmittel aus Dimethylformamid, Dimethylacetamid, N-Methylpyrrolidon oder Dimethylsulfoxid ausgewählt ist.

4. Verfahren nach einem der Ansprüche 1 bis 3, **dadurch gekennzeichnet, dass** die Kondensationsreaktion zwischen dem 4β-Chloracetamido-4'-demethylepipodophyllotoxin und dem primären Aminreaktanten der Formel **6** bei einer Temperatur von 0°C durchgeführt wird.

5. Verfahren nach einem der Ansprüche 1 bis 4, **dadurch gekennzeichnet, dass** auf die Kondensationsreaktion zwischen dem 4β-Chloracetamido-4'-demethylepipodophyllotoxin und dem primären Aminreaktanten der Formel **6** ein Schritt der Gewinnung des Produkts der allgemeinen Formel **1** folgt.

6. Verfahren nach einem der Ansprüche 1 bis 5, **dadurch gekennzeichnet, dass** die Gewinnung der Verbindung der Formel 1 mittels Fällung in einem alkoholischen Lösungsmittel, gefolgt von einer Umkehrphasenchromatographie in einem sauren Medium durchgeführt wird.

7. Verfahren nach einem der Ansprüche 1 bis 6, **dadurch gekennzeichnet, dass** der primäre Aminreaktant der Formel **6,** der in dem Kondensationsschritt mit dem 4β-Chloracetamido-4'-demethylepipodophyllotoxin verwendet wird, Spermin oder Spermidin ist.

8. Verfahren zur Herstellung der Verbindung der Formel **1,** in der a = 3, b = 4 und c = 3, nämlich 2-{3-[4-(3-Aminopropylamino)butylamino]propylamino}-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]acetamid, nach einem der Ansprüche 1 bis 7, **dadurch gekennzeichnet, dass** das Verfahren einen Schritt der Kondensation des 4β-Chloracetamido-4'-demethylepipodophyllotoxins mit dem Spermin umfasst.

9. Verfahren zur Herstellung der Verbindung der Formel **1,** wobei a = 3, b = 4, R = H, nämlich 2-[3-(4-Aminobutylamino)propylamino]-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]acetamid, nach einem der Ansprüche 1 bis 7, **dadurch gekennzeichnet, dass** das Verfahren einen Schritt der Kondensation des 4β-Chloracetamido-4'-demethylepipodophyllotoxins der Formel **4** mit dem Spermidin umfasst.

10. Verfahren zur Herstellung der Verbindung der Formel **1,** wobei a = 4, b = 3, R = H, nämlich 2-[4-(3-Aminopropylamino)butylamino]-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]acetamid, nach einem der Ansprüche 1 bis 7, **dadurch gekennzeichnet, dass** das Verfahren einen Schritt der Kondensation des 4β-Chloracetamido-4'-demethylepipodophyllotoxins der Formel **4** mit dem Spermidin umfasst.

11. Verfahren zur Herstellung von Verbindungen der Formel **1** nach den Ansprüchen 1 bis 7, **dadurch gekennzeichnet, dass** es die folgenden Schritte umfasst:
a) Herstellung des 4'-Demethylepipodophyllotoxins der Formel **3** aus dem Podophyllotoxin der Formel **2** 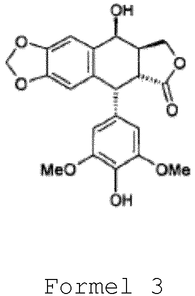 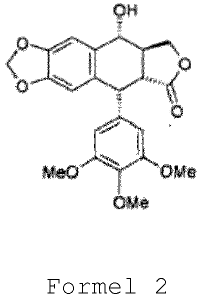
b) Umwandlung des im Schritt a) erhaltenen 4'-Demethylepipodophyllotoxins der Formel **3** in 4β-Chloracetamido-4'-demethylepipodophyllotoxin der Formel **4** 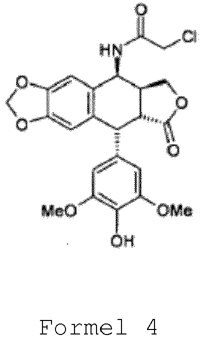
c) Kondensation des im Schritt b) erhaltenen 4β-Chloracetamido-4'-demethylepipodophyllotoxins mit einem primären Aminreaktanten der Formel **6.**

12. Verfahren zur Herstellung der Verbindung der Formel **1**, in der a = 3, b = 4 und c = 3, nämlich 2-{3-[4-(3-Aminopropylamino)butylamino]propylamino}-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]acetamid, nach Anspruch 11, **dadurch gekennzeichnet, dass** das Verfahren die folgenden Schritte umfasst:
a) Herstellung des 4'-Demethylepipodophyllotoxins aus dem Podophyllotoxin
b) Umwandlung des im Schritt a) erhaltenen 4'-Demethylepipodophyllotoxins in 4β-Chloracetamido-4'-demethylepipodophyllotoxin
c) Kondensation des im Schritt b) erhaltenen 4β-Chloracetamido-4'-demethylepipodophyllotoxins mit dem Spermin.

13. Verfahren zur Herstellung der Verbindung der Formel **1**, in der a = 3, b = 4 und R = H, nämlich 2-[3-(4-Aminobutylamino)propylamino]-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]acetamid, nach Anspruch 11, **dadurch gekennzeichnet, dass** das Verfahren die folgenden Schritte umfasst:
a) Herstellung des 4'-Demethylepipodophyllotoxins aus dem Podophyllotoxin
b) Umwandlung des im Schritt a) erhaltenen 4'-Demethylepipodophyllotoxins in 4β-Chloracetamido-4'-demethylepipodophyllotoxin
c) Kondensation des im Schritt b) erhaltenen 4β-Chloracetamido-4'-demethylepipodophyllotoxins mit dem Spermidin.

14. Verfahren zur Herstellung der Verbindung der Formel **1**, in der a = 4, b = 3 und R = H, nämlich 2-[4-(3-Aminopropylamino)butylamino]-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]acetamid, nach Anspruch 11, **dadurch gekennzeichnet, dass** das Verfahren die folgenden Schritte umfasst:
a) Herstellung des 4'-Demethylepipodophyllotoxins aus dem Podophyllotoxin
b) Umwandlung des im Schritt a) erhaltenen 4'-Demethylepipodophyllotoxins in 4β-Chloracetamido-4'-demethylepipodophyllotoxin
c) Kondensation des im Schritt b) erhaltenen 4β-Chloracetamido-4'-demethylepipodophyllotoxins mit dem Spermidin.

15. Verwendung von Verbindungen der allgemeinen Formel 6 zur Herstellung von Verbindungen der allgemeinen Formel 1 nach den Ansprüchen 1 bis 7.

16. Verwendung von Verbindungen der allgemeinen Formel 6 zur Herstellung von Verbindungen der allgemeinen Formel 1 nach den Ansprüchen 11 bis 14.

## Claims

1. Method for preparing (poly)aminoalkylaminoacetamide derivatives of epipodophyllotoxin of formula 1 and the pharmaceutically acceptable salts thereof, wherein R represents a hydrogen atom or a -(CH₂)_{c}-NH₂ group where 2 ≤ a,b,c ≤ 5 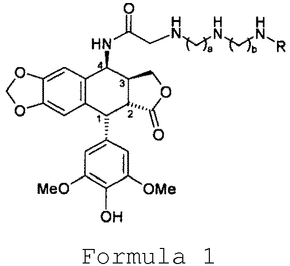 **characterised in that** it comprises a step for condensing 4β-chloroacetamido-4'-demethylepipodophyllotoxin of formula 4 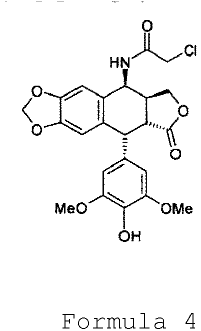 with a primary amine reagent of formula 6: a, b, c and R having the same denotations as above and not including prior protection of the amine functions 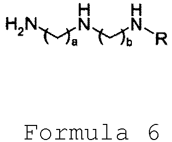 **characterised in that** said condensation step is carried out in a polar aprotic solvent and in a temperature range between -20°C and +30°C, and that said reagents of formula 4 and 6 are in stoichiometric quantities.

2. Method for preparing (poly)aminoalkylaminoacetamide derivatives of epipodophyllotoxin of formula 1 according to claim 1 **characterised in that** they are obtained in hydrochloride form.

3. Method according to any one of claims 1 to 2 **characterised in that** the aprotic polar solvent is chosen from dimethylformamide, dimethylacetamide, N-methylpyrrolidone or dimethylsulphoxide.

4. Method according to any one of claims 1 to 3 **characterised in that** the condensation reaction between 4β-chloroacetamido-4'-demethylepipodophyllotoxin and the primary amine reagent of formula 6 is carried out at a temperature of 0°C.

5. Method according to any one of claims 1 to 4 **characterised in that** the condensation reaction between 4β-chloroacetamido-4'-demethylepipodophyllotoxin and the primary amine reagent of formula 6 is followed by a step for retrieving the product of general formula 1.

6. Method according to any one of claims 1 to 5 **characterised in that** the retrieval of the compound of formula 1 is carried out by precipitation in an alcoholic solvent, followed by reversed-phase chromatography in an acidic medium.

7. Method according to any one of claims 1 to 6 **characterised in that** the primary amine reagent of general formula 6 used during the condensation step with 4β-chloroacetamido-4'-demethylepipodophyllotoxin is spermine or spermidine.

8. Method for preparing the compound of formula 1 wherein a = 3, b = 4 and c = 3, i.e. 2-{3-[4-(3-aminopropylamino)-butylamino]-propylamino}-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]-acetamide according to any one of claims 1 to 7 **characterised in that** it comprises a step for condensing 4β-chloroacetamido-4'-demethylepipodophyllotoxin with spermine.

9. Method for preparing the compound of formula 1 for which a = 3, b = 4, R = H, i.e. 2-[3-(4-aminobutylamino)-propylamino]-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]-acetamide according to any one of claims 1 to 7 **characterised in that** it comprises a step for condensing 4β-chloroacetamido-4'-demethylepipodophyllotoxin of formula 4 with spermidine.

10. Method for preparing the compound of formula 1 for which a = 4, b = 3, R = H, i.e. 2-[4-(3-aminopropylamino)-butylamino]-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]-acetamide according to any one of claims 1 to 7 **characterised in that** it comprises a step for condensing 4β-chloroacetamido-4'-demethylepipodophyllotoxin of formula 4 with spermidine.

11. Method for preparing compounds of formula 1 according to claims 1 to 7 **characterised in that** it comprises the following steps:
a) Preparing 4'-demethylepipodophyllotoxin of formula 3 from podophyllotoxin of formula 2 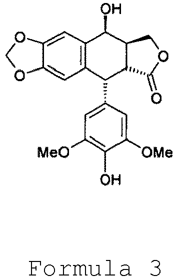 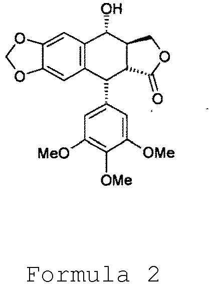
b) Converting the 4'-demethylepipodophyllotoxin of formula 3 obtained in step a) into 4β-chloroacetamido-4'-demethylepipodophyllotoxin of formula 4 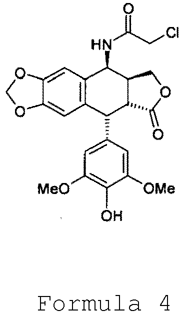
c) Condensing the 4β-chloroacetamido-4'-demethylepipodophyllotoxin obtained in step b) with a primary amine reagent of formula 6.

12. Method for preparing the compound of formula 1 wherein a = 3, b = 4 and c = 3, i.e. 2-{3-[4-(3-aminopropylamino)-butylamino]-propylamino}-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]-acetamide according to claim 11 **characterised in that** it comprises the following steps:
a) Preparing 4'-demethylepipodophyllotoxin from podophyllotoxin
b) Converting the 4'-demethylepipodophyllotoxin obtained in step a) into 4β-chloroacetamido-4'-demethylepipodophyllotoxin
c) Condensing the 4β-chloroacetamido-4'-demethylepipodophyllotoxin obtained in step b) with spermine.

13. Method for preparing the compound of formula 1 for which a = 3, b = 4 and R = H, i.e. 2-[3-(4-aminobutylamino)-propylamino}-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]-acetamide according to claim 11 **characterised in that** it comprises the following steps:
a) Preparing 4'-demethylepipodophyllotoxin from podophyllotoxin
b) Converting the 4'-demethylepipodophyllotoxin obtained in step a) into 4β-chloroacetamido-4'-demethylepipodophyllotoxin
c) Condensing the 4β-chloroacetamido-4'-demethylepipodophyllotoxin obtained in step b) with spermidine.

14. Method for preparing the compound of formula 1 for which a = 4, b = 3 and R = H, i.e. 2-[4-(3-aminopropylamino)-butylamino]-N-[9-(4-hydroxy-3,5-dimethoxyphenyl)-8-oxo-5,5a,6,8,8a,9-hexahydrofuro[3',4':6,7]naphtho[2,3-d][1,3]dioxol-5-yl]-acetamide according to claim 11 **characterised in that** it comprises the following steps:
a) Preparing 4'-demethylepipodophyllotoxin from podophyllotoxin
b) Converting the 4'-demethylepipodophyllotoxin obtained in step a) into 4β-chloroacetamido-4'-demethylepipodophyllotoxin
c) Condensing the 4β-chloroacetamido-4'-demethylepipodophyllotoxin obtained in step b) with spermidine.

15. Use of the compounds of general formula 6 for the preparation of compounds of general formula 1 according to claims 1 to 7.

16. Use of the compounds of general formula 6 for the preparation of compounds of general formula 1 according to claims 11 to 14.
